# EUROPEAN PATENT APPLICATION

(11) **EP 4 563 112 A1**
(43) Date of publication of application: **04.06.2025**
(21) Application number: 24215960.6
(22) Date of filing: 28.11.2024
(51) Int. Cl.: A61B 34/20, A61B 34/10, A61F 2/38, A61F 2/46

(54) **PATELLA TRACKER**

(30) Priority: 01.12.2023 US 202363605023 P
(71) Applicant: Howmedica Osteonics Corporation, Mahwah, New Jersey 07430 (US)
(72) Inventor: Koppaka, Ajay Teja, London, W44AR (GB); Slayton, Alexander, Denville, 07834 (US); Mahadeshwar, Ameya M., 421501 Thane (IN); Ali, Azhar, West Orange, 07052 (US); Tucker, Emerson, Milwaukie, 97222 (US); Mayman, David, New York, 10021 (US)
(74) Representative: Regimbeau

(57) **Abstract**

Disclosed herein are a surgical system for patella tracking and a method for selecting and aligning knee implants utilizing the same. The surgical system may include first and second trackers and a patellar tracking system. The first tracker may be configured to contact an unresected patella, and the second tracker may be configured to contact a bone. The patellar tracking system may be configured to track the first and second trackers during patellar flexion and extension to generate patellar range of motion and patellar trial range of motion. A method for selecting a knee implant may utilize the first and second trackers and the patellar tracking system.

## Description

### FIELD OF INVENTION

The present disclosure relates to systems and methods for sizing and aligning knee implants, and particularly to systems and methods for sizing and aligning knee implants by tracking an unresected patella.

### BACKGROUND OF THE INVENTION

The use of implants for bone treatments requires careful selection of the right implant size to ensure natural biomechanics of the joint. For example, a femoral implant must be properly sized to match the resected femur for optimal postoperative joint kinematics. For a total knee arthroplasty ("TKA") procedure, the femoral implant must be aligned properly to interact with a tibial implant and a patellar implant for proper postoperative joint kinematics. This ensures that the knee joint can move in the same way as it did prior to the surgery or prior to the existence of the disease. If the size of the implant is incorrect and/or is improperly aligned, it can cause unnatural alterations to the joint's movement, resulting in a range of complications.

Choosing the right knee implant to ensure proper knee joint kinematics is a complicated task as the knee range of motion through extension and flexion involves a complex sequence of bone movements comprised of six different degrees of freedom. A single or even multiple knee flexion-extension positions may not be enough to guarantee accurate knee kinematics throughout the entire flexion-extension cycle, and thus the selection of an implant solely on these parameters may not be ideal. A comprehensive assessment of the six degrees of freedom can help in selecting an appropriate knee implant and aligning same to promote natural and successful knee joint movement.

Therefore, there exists a need for systems and methods for sizing and aligning knee implants.

### BRIEF SUMMARY OF THE INVENTION

Disclosed herein are systems and methods for sizing and aligning knee implants. A method according to the present disclosure can be used for tracking and evaluating the size and alignment of tibial and femoral implants. The method may include tracking an unresected patella and displaying a first range of motion through flexion and extension of the knee joint. A bone is then resected, either from the tibia or femur, or both, and the implants are selected and placed. Next, the knee ligament tension is measured. The patella is tracked again, and the second range of motion is displayed. Finally, the two ranges of motion are compared to evaluate the knee ligament tension and corresponding size and alignment of the tibial and femoral implants.

A method according to this aspect provides a systematic way to measure and analyze the size and alignment of tibial and femoral implants during a surgical procedure. By tracking the unresected patella at two points in time, the range of motion is displayed to gain valuable insight into the motion of the knee, enabling the surgeon to measure the tension in the knee ligaments and select the ideal size and alignment of the implants. Consequently, this method allows surgeons to ensure a successful outcome that will provide the patient with maximum comfort and function.

In accordance with an aspect of the present disclosure, a method for sizing knee implants is provided. A method according to this aspect may include the steps of tracking a location of a point adjacent an unresected patella during flexion and extension motion of a knee to generate a first path representing a first patellar range of motion on a display, resecting a tibia and placing a tibial trial or a tibial implant on the resected tibia, detecting a knee ligament tension value, tracking a location of the point adjacent the unresected patella during flexion and extension motion of the knee to generate a second path representing a second patellar range of motion on the display, and displaying and comparing the first path with the second path to evaluate the knee ligament tension value.

Continuing in accordance with this aspect, the method may include a step of adjusting soft tissues of the knee based on the comparison of the first path with second path. The step of adjusting soft tissues of the knee may be performed to obtain a predetermined knee ligament tension value.

Continuing in accordance with this aspect, the step of placing the tibial trial or the tibial implant on the resected tibia may include placing a tibial trial on the resected tibia. The method may further include a step of selecting a tibial implant based on the tibial trial.

Continuing in accordance with this aspect, the point may be located on a surface of the unresected patella.

Continuing in accordance with this aspect, the step of comparing the first path with the second path may include determining a difference between the first path and the second path.

In accordance with another aspect of the present disclosure, a method for sizing knee implants is provided. A method according to this aspect may include the steps of tracking a location of a point adjacent an unresected patella during flexion and extension motion of a knee to generate a first path representing a first patellar range of motion on a display, placing a tibial trial on a tibia, placing a femoral trial on a femur, tracking a location of the point adjacent the unresected patella during flexion and extension motion of the knee to generate a second path representing a second patellar range of motion on the display, and displaying and comparing the first path with the second path to evaluate the tibial trial and the femoral trial.

Continuing in accordance with this aspect, the step of placing the tibial trial may include a step of placing the tibial trial on a resected tibia. The step of placing the femoral trial may include a step of placing the femoral trial on a resected femur. The step of comparing the first path with the second path may include determining a difference between the first path and the second path. The method may include a step of selecting a tibial implant based on the tibial trial if the difference is less than a predetermined threshold. The method may include a step of selecting a femoral implant based on the femoral trial if the difference is less than a predetermined threshold.

Continuing in accordance with this aspect, the point may be located on a surface of the unresected patella.

Continuing in accordance with this aspect, the steps of tracking the location of the point adjacent the unresected patella in flexion and extension may include the step of placing a first tracker on the patella and a second tracker on a bone and using a patellar tracking system to generate the first and second paths representing the patellar range of motion with reference to the bone. The step of placing a first tracker on the unresected patella may include the step of placing a probe on a check post in contact with the unresected patella. The patellar range of motion may be generated by the step of registering the position of the first tracker on the unresected patella with reference to the bone in at least a first, a second and a third position. The first position may be at patellar flexion. The third position may be at patellar extension and a second position may be located therebetween. The step of registering may include using a tracking camera to register the positions of the first and second trackers.

Continuing in accordance with this aspect, the step of displaying and comparing the first path with the second path may include displaying and comparing the first path and the second path on a display screen.

In accordance with an aspect of the present disclosure, a system for sizing knee implants is provided. A system according to this aspect may include a tracker configured to track a location of a point adjacent an unresected patella during flexion and extension motion of a knee to generate a path representing a patellar range of motion, a resection tool configured to resect a tibia, a tibial trial or a tibial implant configured to be placed on the resected tibia, a ligament tension detector to detect a knee ligament tension value, and a display configured to display and comparing a first path of the patellar range of motion with a second path of the patellar range of motion to evaluate the knee ligament tension value. The first path may be generated prior to resecting the tibia and the second path may be generated after resecting the tibia.

Continuing in accordance with this aspect, the tracker may comprise a probe configured to contact the unresected patella and a second tracker positioned on a bone to generate the patellar range of motion relative to the bone.

Continuing in accordance with this aspect, the tracker may include a tracking camera configured to register a position of the patella at multiple angles of flexion and extension of the knee.

Continuing in accordance with this aspect, the ligament tension detector may include load sensors configured to measure ligament tension during the range of motion.

Continuing in accordance with this aspect, the resection tool may be further configured to resect a femur. The system may further include a femoral trial or a femoral implant configured to be placed on a resected femur.

Continuing in accordance with this aspect, the system may include a computing module configured to calculate a difference between the first path and the second path of the patellar range of motion.

Continuing in accordance with this aspect, the display may be configured to overlay the first path and the second path on a common coordinate system for visual comparison.

Continuing in accordance with this aspect, the system may include an adjustment tool configured to adjust soft tissues of the knee to achieve a predetermined knee ligament tension value based on a comparison of the first and second paths.

Continuing in accordance with this aspect, the tracker may be configured to generate the patellar range of motion using at least three discrete patellar positions. The three discrete patellar positions may include a flexion position, an extension position, and an intermediate position.

Continuing in accordance with this aspect, the ligament tension detector may include a database configured to store knee ligament tension values corresponding to patient profiles for comparison.

Continuing in accordance with this aspect, the tibial trial or the tibial implant may include adjustable components for varying implant size or alignment during placement.

Continuing in accordance with this aspect, the tibial trial or tibial implant may include sensors configured to monitor alignment and pressure distribution during placement.

Continuing in accordance with this aspect, the tracker may be configured to track motion across multiple flexion-extension cycles and generate an averaged path for the first and second patellar range of motion.

### BRIEF DESCRIPTION OF THE DRAWINGS

A more complete appreciation of the subject matter of the present disclosure and the various advantages thereof can be realized by reference to the following detailed description, in which reference is made to the following accompanying drawings:
FIG. 1 is a flowchart showing the steps for sizing and aligning a tibial implant according to an embodiment of the present disclosure;
FIG. 2 is a flowchart showing the steps for sizing and aligning a femoral implant according to an embodiment of the present disclosure;

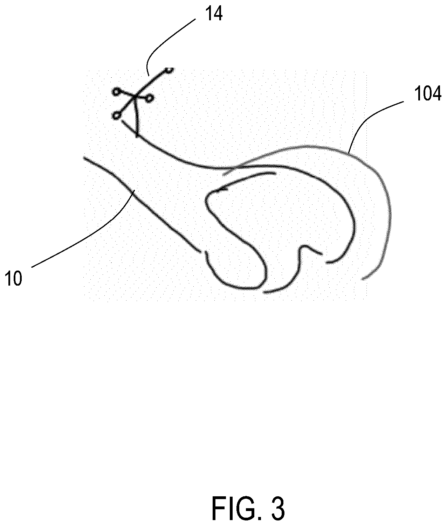

FIG. 3 is a schematic view showing a first patellar range of motion according to an embodiment of the present disclosure;
FIG. 4 is a schematic view showing a second patellar range of motion according to an embodiment of the present disclosure;
FIG. 5 is a schematic view of a patellar implant tracking display according to an embodiment of the present disclosure;
FIG. 6 is a sagittal view of the patellar implant tracking of FIG. 5;
FIG. 7 is a perspective view of a patellar marker according to an embodiment of the present disclosure;
FIG. 8 is a perspective view of a patellar marker according to another embodiment of the present disclosure;
FIG. 9A is a perspective view of a ring of a patellar marker according to another embodiment of the present disclosure;
FIG. 9B is a perspective view of a base of the patellar marker of FIG. 9A;
FIG. 10A is a perspective view of a patellar marker according to another embodiment of the present disclosure;
FIG. 10B is a side view of the patellar marker of FIG. 10A;
FIG. 10C is a cross-sectional view of the patellar marker of FIG. 10A;
FIG. 11 is a front view of a patellar marker according to another embodiment of the present disclosure, and
FIG. 12 is a front view of a patellar marker according to another embodiment of the present disclosure.

### DETAILED DESCRIPTION

Reference will now be made in detail to the various embodiments of the present disclosure illustrated in the accompanying drawings. The term "a," as used in the specification, means "at least one." The terminology includes the words above specifically mentioned, derivatives thereof, and words of similar import. Although at least two variations are described herein, other variations may include aspects described herein combined in any suitable manner having combinations of all or some of the aspects described. Wherever possible, the same or like reference numbers will be used throughout the drawings to refer to the same or like features within a different series of numbers (e.g., 100-series, 200-series, etc.)

In describing preferred embodiments of the disclosure, reference will be made to directional nomenclature used in describing the human body. It is noted that this nomenclature is used only for convenience and that it is not intended to be limiting with respect to the scope of the invention. As used herein, when referring to bones or other parts of the body, the term "anterior" means toward the front part or the face and the term "posterior" means toward the back of the body. The term "medial" means toward the midline of the body and the term "lateral" means away from the midline of the body. The term "superior" means closer to the heart and the term "inferior" means more distant from the heart.

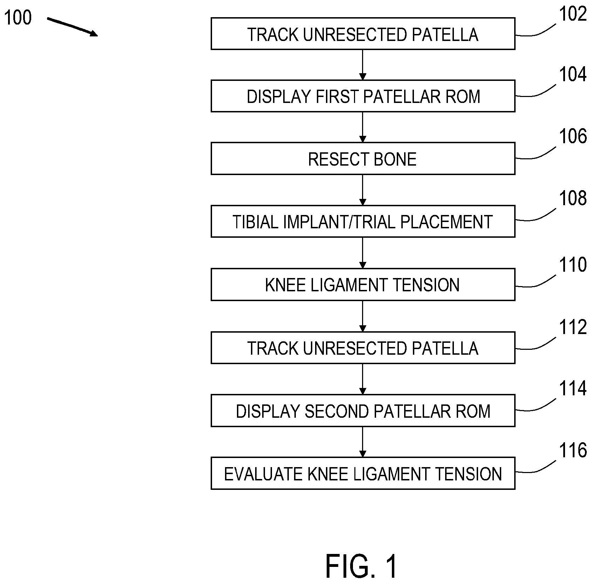

FIG. 1 is a flowchart 100 showing steps for sizing and aligning a tibial implant according to an embodiment of the present disclosure. An unresected patella is dynamically tracked in a step 102. A tracking system with a position capturing device and a display can be used to track the unresected patella during a knee range of motion. The trackers can include one or more trackers positioned relative or on the unresected patella. For example, a tracker with a distal tip configured to contact the bone and a plurality of fiducials at a proximal end can be located on the femur. The fiducials can be spherical-shaped markers which can be identified by a position capturing device. The number, shape, and properties of the markers can depend on the type of the position capturing device and the nature of the bone being tracked. For example, radiopaque markers can be used with an X-ray or a CT scan position capturing device, while markers with reflectors can be used with a camera position capturing device.

Distinct points are selected on the unresected patella with a probe at different flexion-extension angles of the knee joint in step 102. The tracking system then generates an arc of motion of the patella based on the selected points in a step 104. This arc is defined by a continuous line that tracks the unresected patella throughout the extension-flexion movement of the knee joint. The tibia is then resected in a step 106 based on preoperative or intraoperative resection depth calculation. A tibial trial or implant is then selected and positioned in a step 108. The selection and placement of the tibial trial or implant can be a highly individualized process, based on an array of factors, both pre- and intraoperative. These may include the patient's demographic details such as age, lifestyle, and ethnicity, as well as anatomical features such as bone density, the intended end result, the anticipated lifestyle for the implant, and the patient's preference. In addition, the size and type of tibial implant to be used can impact the selection and placement process, as can the patient's medical history and lifestyle. The preoperative or intraoperative plan is designed to consider all of these factors in order to ensure the best surgical outcome and the most aesthetically pleasing long-term result for the patient.

Knee ligament tension is then measured using load sensors or other suitable means in a step 110. Ligament release can be performed in order to achieve the desired tension for the implant trial. Precision is critical, as the tension applied to the knee ligament may determine the tibial implant's range-of-motion and stability. Accurate calculations are used to determine the desired tension, such as preoperative or intraoperative assessments. Alternatively, the desired ligament tension can be determined through a database that factors in aspects such as the patient's age and gender, the trial implant size, and the targeted range-of-motion for the implant to determine the optimal ligament tension.

In a step 112, the unresected patella is tracked again through its range of motion while the tibial trial or tibial implant is placed upon the resected tibia. The system then generates a second arc of motion of the unresected patella with the tibial trial or implant in place and then displays both the first arc of motion of the unresected patella and the second arc of motion on a display screen in a step 114. The comparison of the two arcs of motion allows for an evaluation of the suitability of tibial trial or implant. Variations to this concept may include tracking the motion of the knee over multiple repetitions of the arc of motion.

In a step 116 of the process, the knee ligament tension is evaluated by comparing the first and second arcs of motions of the unresected patella. This comparison provides important information about the tension of the knee ligaments across the range of motion, which can be used to troubleshoot issues with the size and/or placement of the tibial trial or implant. For example, if the distance between the first and second arc remains constant as the knee goes through its full range of motion, this could indicate that the tibial trial or implant is properly sized and placed. If there are variations in the distance between the two arcs, this can indicate that the tibial trial is improperly sized and/or positioned.

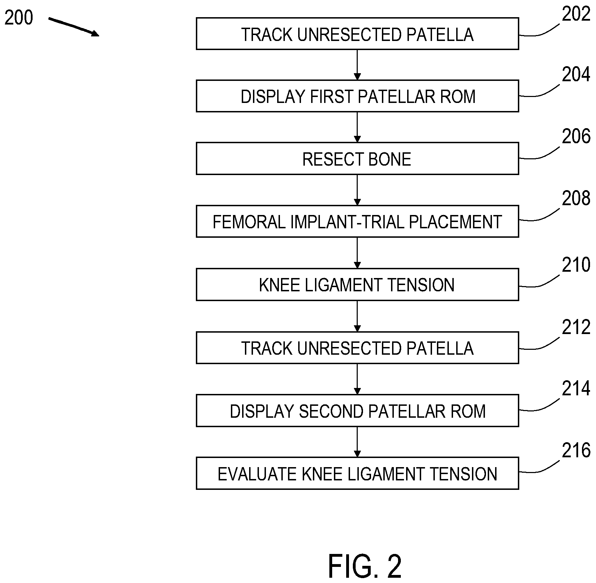

FIG. 2 shows a flowchart 200 detailing steps for sizing and aligning a femoral implant according to an embodiment of the present disclosure. Flowchart 200 is similar to flowchart 100, and therefore like method steps are referred to with similar numerals within the 200-series. An unresected patella can be dynamically tracked using a tracking system in a step 202. This system typically consists of a position capturing device and an associated display. The tracking system can have one or more trackers located on or relative to the unresected patella. Generally, a tracker is configured to have a distal tip that contacts the bone as well as a plurality of fiducials at its proximal end. The fiducials can be spherical-shaped markers which are identified by the position capturing device. The nature and variety of these markers may vary depending on the type of the position capturing device and the bone being tracked. For example, radiopaque markers can be used with an X-ray or a CT scan position capturing device, while markers with reflectors can be used with a camera position capturing device.

Points are specifically chosen on the unresected patella when the knee joint is at different levels of flexion-extension in step 202. The tracking system then creates an arc to track and monitor the unresected patella during the movement from extension to flexion in a step 204. This arc consists of a continuous line which tracks the movements of the unresected patella. Afterwards, in step a 206, the femur is resected according to a preoperative or intraoperative plan for this depth calculation. In a step 208, a selection is made for a femoral trial or implant to be positioned. In order to achieve the best surgical result and a long-term outcome, the selection and placement process of the femoral implant can be based on various preoperative and intraoperative factors like the patient's age, lifestyle, ethnicity, bone density, and medical history. In addition, the size and type of implant to be used will impact the selection and placement process. These factors are all taken into consideration when preoperatively or intraoperatively creating a plan in order to generate the best outcome.

In a step 210, knee ligament tension is measured using load sensors or other suitable means. Ligament release can be performed to achieve the desired tension for the implant as the tension applied to the knee ligament impacts the femoral implant's range-of-motion and stability. To determine the optimal tension, computer calculations can be used to consider factors like the patient's age and gender, the trial implant size, and the targeted range-of-motion for the implant. Alternatively, a database can be consulted to obtain the desired ligament tension based on these criteria.

In a step 212, the unresected patella is tracked through its range of motion while the femoral trial or implant is positioned on the resected femur. The system then generates a second arc of motion of the unresected patella when the femoral trial or implant is in place and displays both arcs of motion on a display screen at a step 214. This enables comparison of the two arcs of motion to evaluate the suitability of the femoral trial or implant. Additionally, it is possible to track the motion of the knee over multiple replays of the arc of motion.

In a step 216, the tension of the knee ligaments is evaluated by comparing the distances between the first and second arcs of motion of the unresected patella. This comparison provides important data about the tension of the knee ligaments across its range of motion, enabling an assessment of the size and/or placement of the femoral trial or implant. If the distance between the first and second arcs remains constant throughout the knee's full range of motion, this indicates that the femoral trial or implant is correctly sized and placed. Deviations from this consistency indicate that the femoral trial is too small or large for the joint, and/or improperly positioned. While a tibial implant or trial is described in flowchart 100 and a femoral implant or trial is described in flowchart 200, in another embodiment both tibial and femoral implants can be sized and positioned on the resected tibia and femur, respectively, using the unresected patella tracking arcs.

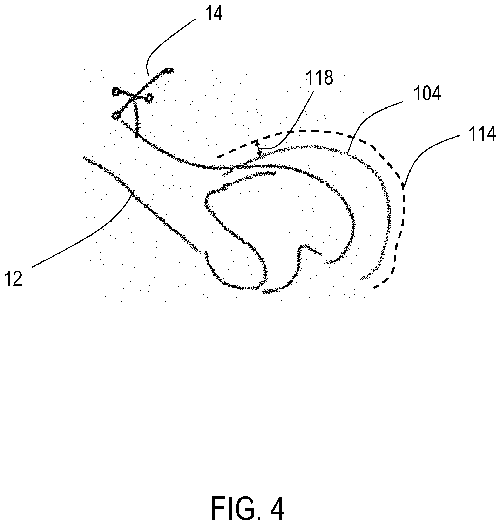

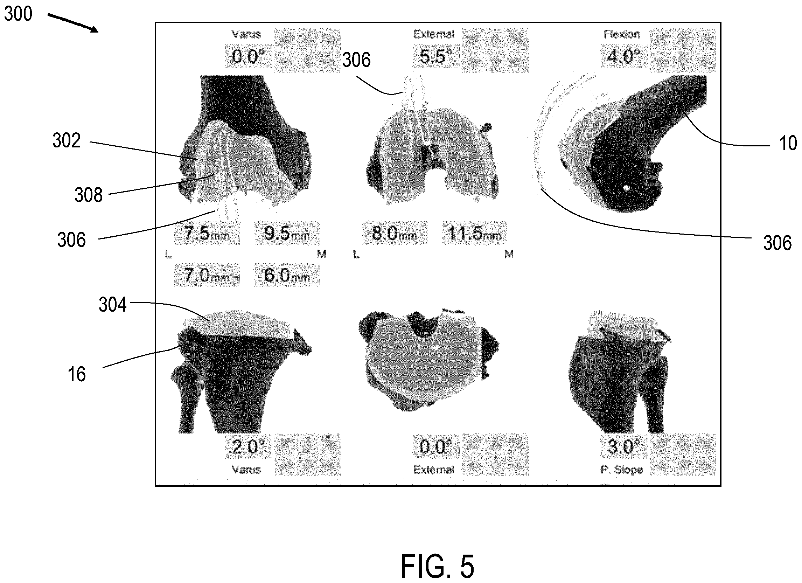

FIG. 4 shows a tracked first patellar range of motion 104 with respect to a femur 10 according to an embodiment of the present disclosure. A femoral array 14 positioned on femur 10 can be used to identify the location of the joint and joint range of motion during tracking and recording of the first patellar range of motion. A probe such as a patellar implant tracker described below can be positioned on the unresected patella or adjacent the unresected patella at a fixed location to ensure the probe is firmly secured during a joint range of motion. Multiple recordings of the probe can be taken during a joint range of motion to identify and generate a trajectory line of the first patellar range of motion 104. Similarly, a second patellar range of motion 114 can be obtained after resection and/or placement of a trial or implant on the resected femur or tibia. As shown in FIG. 5, first patellar rang of motion 104 and second patellar range of motion 114 can be displayed and compared with respect to femur 12 or other joint features to evaluate surgical workflow steps. A difference 118 between the first and second patellar range of motion can be used to evaluate and optimize bone resection depth, trial or implant size and placement. Difference 118 can also aid in the determination of ligament balancing.

Referring now to FIG. 5, there is shown a patellar tracking display 300 of the tracking system according to an embodiment of the present disclosure. Patellar tracking display 300 includes sagittal, coronal, and transverse views of a patient's knee joint. A femoral implant or trial 302 and a tibial implant or trial 304 are shown with patellar tracking information generated by tracking an unresected patella. The tracking system allows users to initiate and terminate a recording of the trajectory of a tracked probe associated with the unresected patella. Tracking and recording of the unresected patella can be based on the different stages of the surgical workflow. For example, this can be done by selecting one of the following options that correspond with the specific steps in the surgical process: 1) initial assessment before resection, 2) tensioning after the tibia is resected but before the femur is resected, and 3) trialing after both the tibia and femur are resected. These options align with the actual steps that take place during the surgical procedure and allow for more accurate organization and analysis of the recorded data.

When the tracking process is initiated, a visual marker such as a red sphere with a 1mm radius can be presented to the user indicating the most recently recorded probe location. This can be accompanied by a live tracked model of the probe and a distinct audible sound, signaling that a new position of the unresected patella had been captured. Throughout the recording, the sphere's position can be updated as the probe-tip's location changes. The user's primary task during the recording is to focus on the bending and straightening of the tibia in relation to the femur while the tracking system generates the patella trajectory.

The location of the probe serves as the patella tracker position is recorded in relation to the knee flexion angle during the data collection process. This can be done in increments of 5 degrees of flexion, resulting in a more precise and accurate measurement. For instance, if the user performed multiple passes of flexion and extension, five patella positions would be recorded while the knee was within the range of 20-25 degrees of flexion. These 5 positions can then be averaged together, producing a single, average point that represents a 20-25-degree flexion value.

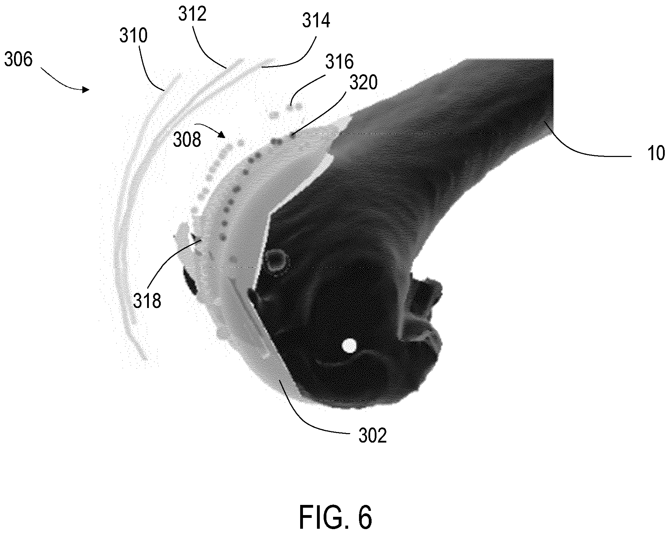

After the patella trajectory recording has concluded, the results can be presented to the user via patellar tracking display 300 as shown in FIGS. 5 and 6. The results can include multiple visuals that allow for comparison between different trajectories. For example, a first set of visual displays can show the average probe positions versus flexion angle trajectory line 310, 312, 314 in relation to a femur bone model 10. Three average probe positions 310, 312, 314 are shown in FIGS. 5 and 6 indicating three recordings of the unresected patellar range of motion. All three recordings are shown simultaneously on display 300, which provides a convenient opportunity to compare their results allowing the user to observe the movement of the unresected patella relative to femur bone model 10 under various conditions such as pre-resection, post-resection, ligament tensioning, implant or trial sizing and placement.

A second set of visuals calculates the closest point 308, 316, 320 between each vertex of the trajectory line 310, 312, 314 and either the femur bone mesh model 10 or a femur implant or trial mesh model 302 as shown in FIGS. 5 and 6. These values can vary depending on the step of the knee surgery. For the initial assessment and tensioning steps, the trajectory uses the probe to the femur bone 10 surface as a reference point, as the femoral implant 302 has not yet been placed. However, for a trialing step, the trajectory uses the planned implant 302 surface as the reference point, as the implant is virtual at this stage and can be adjusted by the user.

A third set of visuals can display an average distance between each vertex on the unresected patella trajectory line 310, 312, 314 and the mesh surface. This distance can be calculated for each recording and provides insight into the potential looseness or tightness of the newly planned or trialed implant in relation to the initial state of the knee. Thus, a position of the femoral trial or implant can be varied to generate new visuals that can be compared and assessed to optimize implant size and position. Thus, the tracking system allows for multiple passes of knee flexion-extension to be completed, ensuring a comprehensive and thorough analysis. Additionally, for each position within the joint range of motion, an average patella trajectory point can be calculated by combining the previously averaged probe locations. These points are then connected by a 3D line, creating a visual representation of the patella trajectory within that specific flexion range as shown in FIGS. 5 and 6. A user can choose whether to display or hide multiple trajectory lines and the closest identified points of bones or implants, allowing for a comprehensive comparison of the knee joint using the unresected patella tracking information.

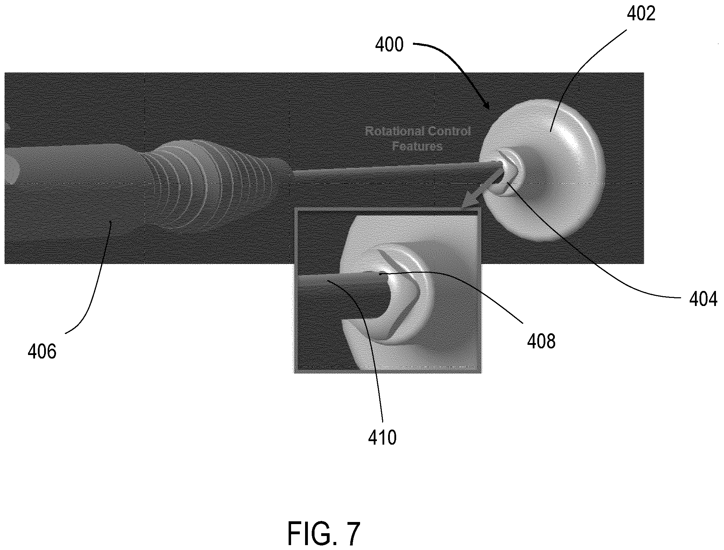

FIG. 7 is a perspective view of a patellar probe 406 with a patellar marker 400 according to an embodiment of the present disclosure. Patellar marker 400 can be used to track the movement of an unresected patella during surgery. Patellar marker 400 includes a proximal surface 402 to specifically fit and interact with patellar probe 406. A groove 408 on proximal surface 402 acts as a rotational control mechanism, providing stability during the surgical procedure. Additionally, patellar probe 406 includes a ridge 410 along its length that is specifically crafted to fit into groove 408 of patellar marker 404 as shown in FIG. 7. A distal end of patellar marker 400 includes one or more engagement features such as pointed edges (not shown) to allow it to securely attach to an unresected patella, allowing for accurate tracking and measurement of its movement.

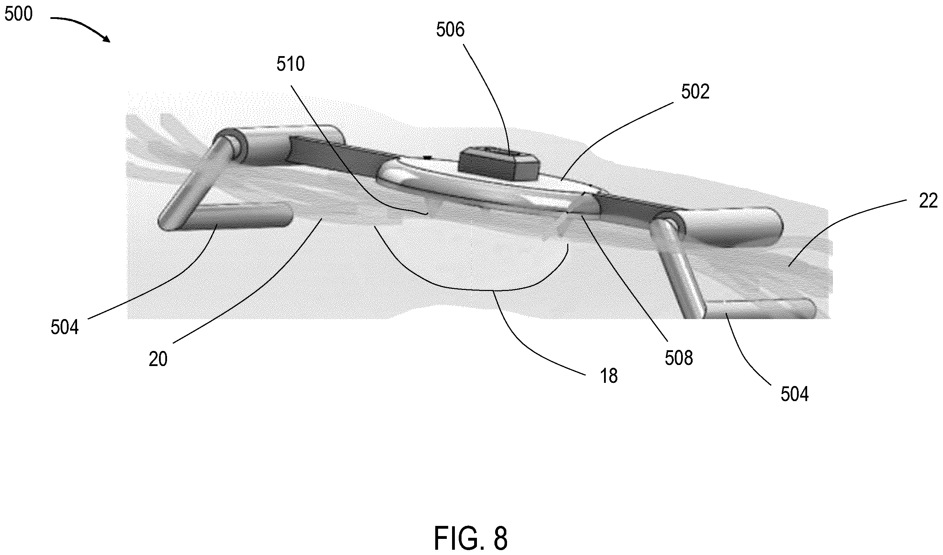

Referring now to FIG. 8, there is a shown a patellar marker 500 according to another embodiment of the present disclosure. Patellar marker 500 includes a base with a proximal surface 502 with an opening 506 to receive and secure a probe therein. A distal surface 508 of the base includes one or more engagement features such as teeth 510 to secure patellar marker 500 to an anterior surface of an unresected patella 18 as shown in FIG. 8. Patellar marker 500 has first and second ligament holders 504 extending from opposite ends of the patellar marker to receive and secure quadriceps 20 and patella tendons 22 and thereby providing a stable foundation for the patellar marker during the joint range motion.

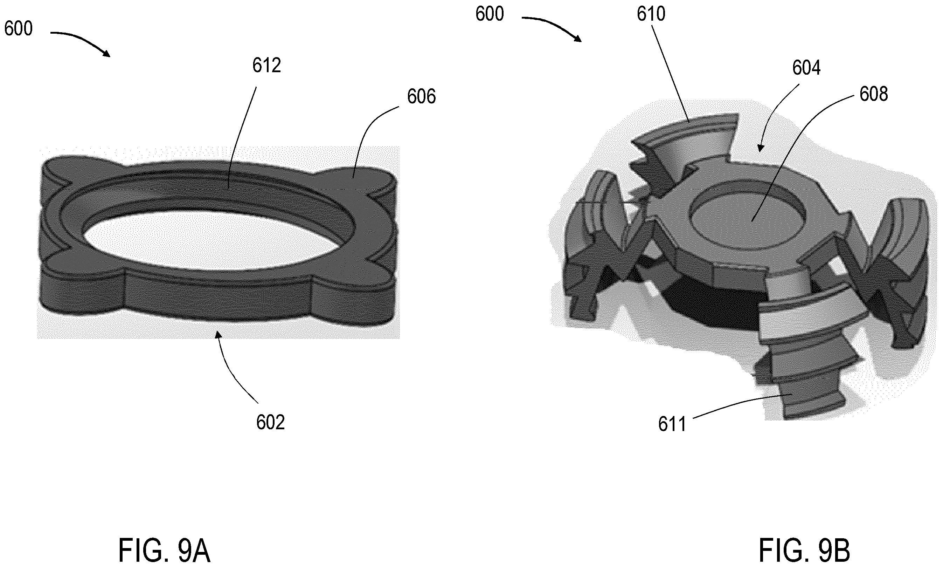

FIGS. 9A and 9B show a patellar marker 600 according to another embodiment of the present disclosure. Patellar maker includes a ring 606 (FIG. 9A) and a base 604 (FIG. 9B). Base 604 has arms 610, each equipped with external threads 611 on an outside surface and a holding pattern 608 on the inside. The ring, on the other hand, features an internal thread 612 that can be rotated to engage with external threads 611 on the arms, resulting in a squeezing action on the soft tissues surrounding the patella bone providing reliable fixation of the patella bone. Arms 610 can secure the patella bone with two points on the medial side and one point on the lateral side, effectively pinching the patella bone from both sides thereby increasing securement and stability of the patellar marker during knee joint motion. Further, as no bone penetrations are required to secure patellar maker 600 to a patella, this design provides for better healing of the patella bone.

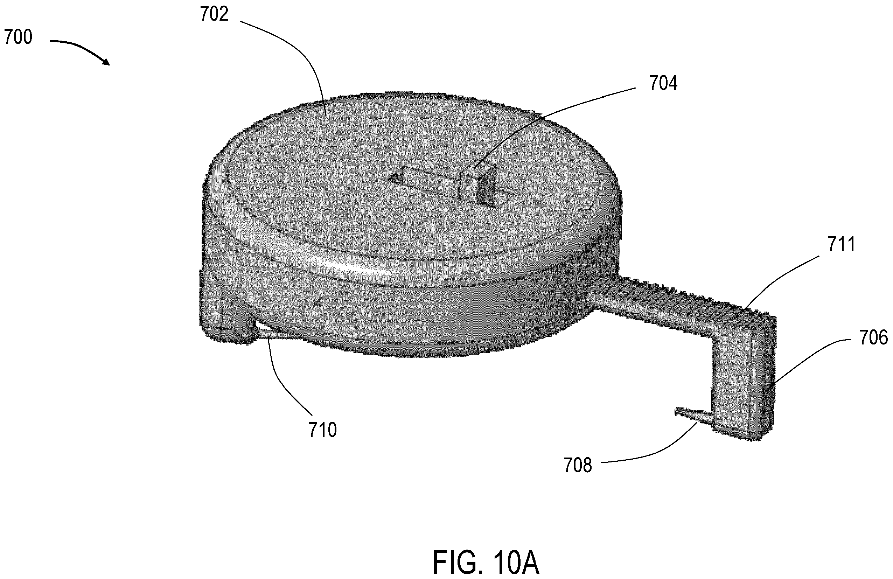

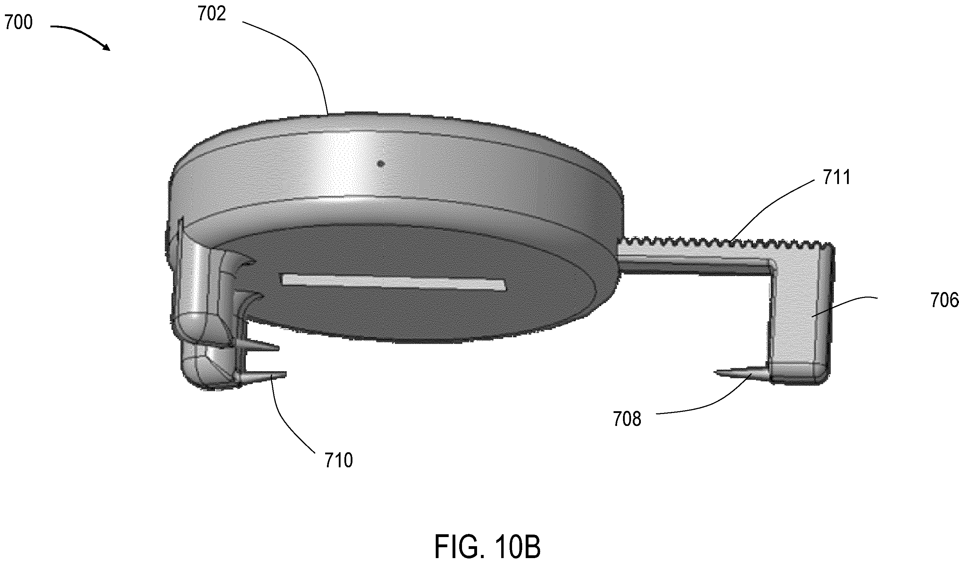

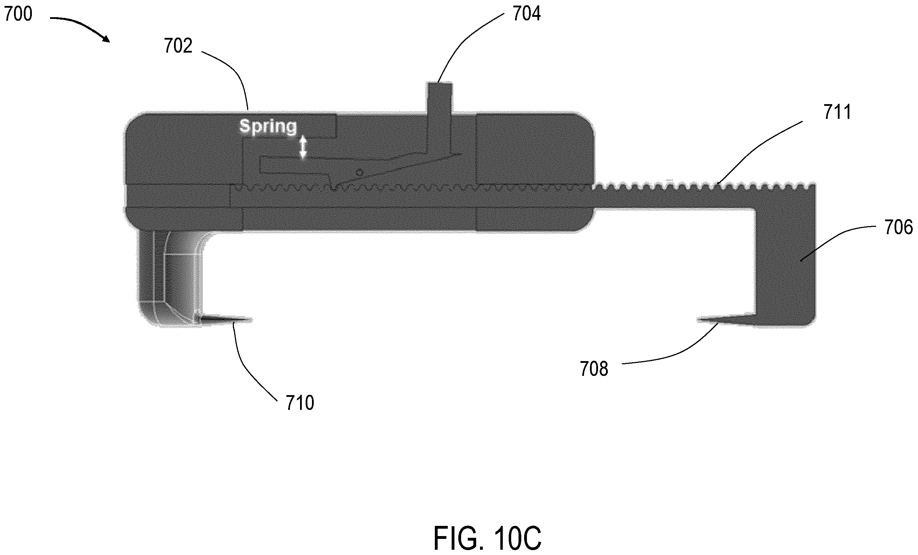

A patellar marker 700 according to another embodiment of the present disclosure is shown in FIGS. 10A-10C. Patellar marker 700 includes a base 702 and a sliding arm 706. The base of patellar marker 700 is made of a circular plate, providing a stable and flat surface on which the device rests on the anterior surface of the soft tissue over the patella. This circular plate can be adjusted to fit comfortably and securely over the patella, ensuring stability during the procedure. Two pointed prongs 710 on the base allow it to firmly dig into the side of the patella, preventing any movement or displacement during tracking the patellar range of motion. Sliding arm 706 of patellar marker 700 includes another pointed prong 708, strategically designed to firmly secure the patella in place. Prong 708 works in conjunction with prongs 710 on base 702, creating a strong grip over the patella for secure and precise positioning. Sliding arm 706 includes a rack and pinion mechanism 711 that allows the arm to be easily and precisely moved towards or away from the patella, ensuring accurate placement and alignment. A release button 704 is located on a proximal surface of base 702, allowing the user to easily disengage patellar marker 700 from the patella. This release button activates the rack and pinion mechanism on the sliding arm, allowing for quick and effortless removal without compromising the accuracy of the positioning. Patellar marker 700 provides an accurate and precise patella position capturing marker with its stable base, secure prongs, adjustable sliding arm, and convenient release mechanism.

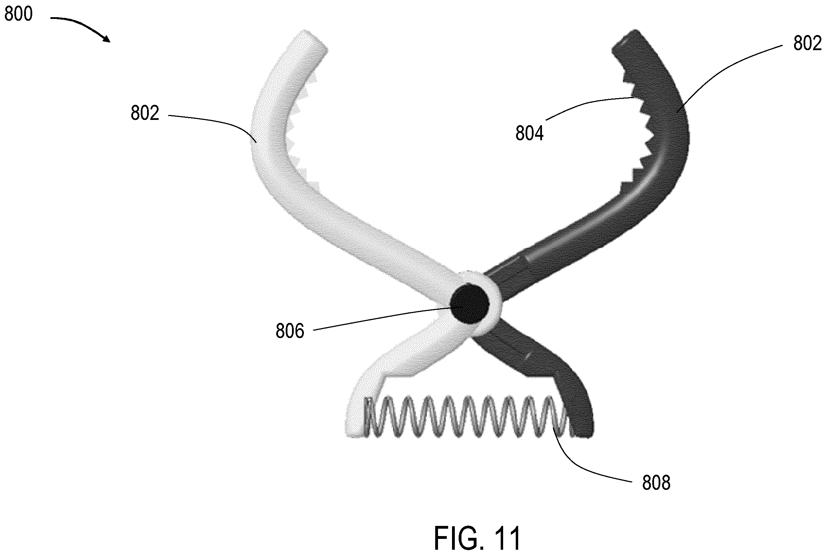

FIG. 11 shows a patellar marker 800 according to another embodiment of the present disclosure. Patellar marker 800 includes two arms 802 with teeth 804 on the inner surface, designed to effectively grip and dig into the sides of the patella. The arms are also equipped with a compression spring 808, ensuring a secure and stable hold on the patella. To use patellar marker 800, an operator simply needs to pinch the arms with their thumb and forefinger to open them up. They can then place the arms around the patella, and once the force is removed, the compression spring will activate and firmly secure the patella marker in place. A joining pin 806 between the arms of the patellar marker 800 includes a hollow feature, which serves as a receiver for a probe. This allows the operator to attach the patellar marker to the probe and use it for accurate and precise patellar range of motion tracking.

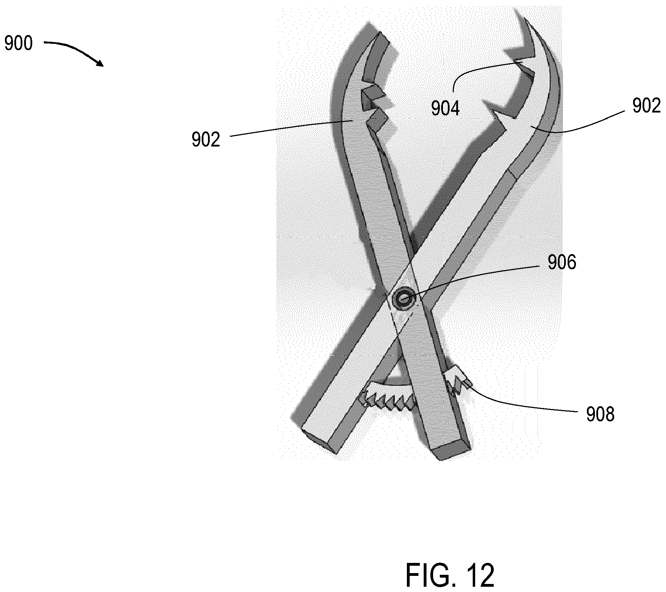

FIG. 12 shows a patellar marker 900 according to another embodiment of the present disclosure. Patellar marker 900 is similar to patellar marker 800, and therefore like elements are referred to with similar numerals within the 900-series of numbers. For example, patellar maker 900 includes first and second arms 902 with teeth 904 on the inner surface. However, patellar marker 900 includes a locking mechanism 908 utilizing a circular rack and pinion mechanism instead of a compression spring used by patellar marker 800. This allows for a secure and precise lock, providing increased stability and accuracy in tracking the patellar position. The use of a circular rack and pinion mechanism also makes it convenient to adjust and fine-tune the patellar marker's position, ensuring optimal placement for the most accurate results.

Furthermore, although the invention disclosed herein has been described with reference to particular features, it is to be understood that these features are merely illustrative of the principles and applications of the present invention. For example, the systems and methods for sizing and aligning knee implants disclosed herein can be performed by tracking a resected patella. It is therefore to be understood that numerous modifications, including changes in the sizes of the various features described herein, may be made to the illustrative embodiments and that other arrangements may be devised without departing from the spirit and scope of the present invention. In this regard, the present invention encompasses numerous additional features in addition to those specific features set forth in the paragraphs below. Moreover, the foregoing disclosure should be taken by way of illustration rather than by way of limitation as the present invention is defined in the examples of the numbered paragraphs, which describe features in accordance with various embodiments of the invention, set forth in the paragraphs below.

## Claims

1. A system for sizing knee implants comprising:
a tracker configured to track a location of a point adjacent an unresected patella during flexion and extension motion of a knee to generate a path representing a patellar range of motion;
a resection tool configured to resect a tibia,
a tibial trial or a tibial implant configured to be placed on the resected tibia;
a ligament tension detector to detect a knee ligament tension value, and
a display configured to display and comparing a first path of the patellar range of motion with a second path of the patellar range of motion to evaluate the knee ligament tension value, wherein the first path is generated prior to resecting the tibia and the second path is generated after resecting the tibia.

2. The system of claim 1, wherein the tracker comprises a probe configured to contact the unresected patella and a second tracker positioned on a bone to generate the patellar range of motion relative to the bone.

3. The system of claim 1, wherein the tracker includes a tracking camera configured to register a position of the patella at multiple angles of flexion and extension of the knee.

4. The system of any one of claims 1 to 3, wherein the ligament tension detector comprises load sensors configured to measure ligament tension during the range of motion.

5. The system of any one of claims 1 to 4, wherein the resection tool is further configured to resect a femur.

6. The system of claim 5, further comprising a femoral trial or a femoral implant configured to be placed on a resected femur.

7. The system of any one of claims 1 to 6, further comprising a computing module configured to calculate a difference between the first path and the second path of the patellar range of motion.

8. The system of any one of claims 1 to 7, wherein the display is configured to overlay the first path and the second path on a common coordinate system for visual comparison.

9. The system of any one of claims 1 to 8, further comprising an adjustment tool configured to adjust soft tissues of the knee to achieve a predetermined knee ligament tension value based on a comparison of the first and second paths.

10. The system of any one of claims 1 to 9, wherein the tracker is configured to generate the patellar range of motion using at least three discrete patellar positions.

11. The system of claim 10, wherein the three discrete patellar positions include a flexion position, an extension position, and an intermediate position.

12. The system of any one of claims 1 to 11, wherein the ligament tension detector includes a database configured to store knee ligament tension values corresponding to patient profiles for comparison.

13. The system of any one of claims 1 to 12, wherein the tibial trial or the tibial implant includes adjustable components for varying implant size or alignment during placement.

14. The system of any one of claims 1 to 13, wherein the tibial trial or tibial implant includes sensors configured to monitor alignment and pressure distribution during placement.

15. The system of any one of claims 1 to 14, wherein the tracker is configured to track motion across multiple flexion-extension cycles and generate an averaged path for the first and second patellar range of motion.
